# EUROPEAN PATENT APPLICATION

(11) **EP 3 821 809 A1**
(43) Date of publication of application: **19.05.2021**
(21) Application number: 19208751.8
(22) Date of filing: 13.11.2019
(51) Int. Cl.: A61B 6/00, A61B 6/08

(54) **PATIENT POSITIONING METHOD FOR RADIOGRAPHIC IMAGING**

(71) Applicant: AGFA NV, 2640 Mortsel (BE)
(72) Inventor: EXELMANS, Walter, 2640 Mortsel (BE)
(74) Representative: Verbrugghe, Anne Marie L.

(57) **Abstract**

Method of positioning a body region of a patient for a second radiographic image recording on the basis of a fluoroscopic image of the same body region or part thereof taken for positioning the patient for a first radiographic image recording.

## Description

### FIELD OF THE INVENTION

The present invention relates to a method of digital x-ray image recording.
In particular, the invention relates to a method for positioning a body or body region for radiographic image recording in a radiographic system with the aid of a fluoroscopic image or a series of fluoroscopic images.

### BACKGROUND OF THE INVENTION

X-ray imaging is a non-invasive technique to capture medical images of patients or animals as well as to inspect materials or the contents of certain containers such as luggage, packages etc.

Image recording comprises irradiating an object with an X-ray beam. As the X-ray beam passes through the object, it is attenuated by a degree that varies with the internal composition and/or thickness of the object. An X-ray detector is then arranged to capture the image-wise modulated X-ray beam and to convert the image-wise modulated X-ray beam into an image showing the internal structure of the object.

In the field of radiographic image recording it is nowadays common to use re-usable radiography detectors (also called 'panels', 'flat panels', 'flat panel detectors' etc.) for X-ray image recording.

Different types of such re-usable detectors exist.

One type of X-ray recording devices used in so-called computed radiography (CR) comprises a photo-stimulable phosphor detector which stores image-wise modulated radiation when it is exposed to an X-ray image.

To release the stored image, the photostimulable phosphor detector is scanned by means of stimulating radiation, e.g. by means of laser light of an appropriate wavelength adapted to the type photostimulable phosphor.

Upon stimulation the phosphor detector releases the stored radiation image in the form of image-wise modulated light which is then detected by a photo-electric convertor so as to generate an electric signal representation of the irradiated object.

This electric signal representation can then be processed in a signal processor and / or stored. It can further be applied to a display device or to a printer to generate a visible image on which an evaluation, e.g. a diagnosis in case of a medical image, can be performed.

Increasingly, radiography flat panel detectors (FPD) for direct radiography (DR) are being used which can convert X-rays in a direct way (direct conversion direct radiography), or in an indirect way (indirect conversion direct radiography) into an electric signal representation.

In direct radiography, the radiography detectors convert X-rays directly into electric charges directly interacting with a photoconductive layer such as amorphous selenium (a-Se). In indirect conversion direct radiography, the radiography flat panel detectors have a scintillator layer consisting of CsI:Tl or Gd2O2S which converts X-rays into light which then interacts with the sensor being an amorphous silicon (a-Si) semiconductor layer, where electric charges are created.

In both systems settings of the source of radiation relative to the support of the patient subject to irradiation have to be performed prior to the recording of an x-ray image.

Settings have to be performed regarding the dose that will be emitted by the source of radiation as well as regarding the relative positioning of the patient and the source of radiation (location, source-patient distance ...) .
Further, collimator settings may be necessary to limit the beam of radiation to a specific area on the patient's body where the radiation is intended to impinge.

It may happen that the positioning of the object (patient) is not optimal. The positioning is usually evaluated on a recorded x-ray image by a radiographer based on his experience or on his perception of the radiographic image.

However, if the positioning was incorrect, the exposure has to be repeated. This implies more dose for the patient which may be harmful, more time to spent by the radiographer and extended occupation of the radiography system.

It is common practice to guide the positioning of a patient for radiographic image recording by means of a fluoroscopic image or a series of fluoroscopic images of the patient in a position intended for radiographic image recording.

Fluoroscopic images are images generated by emitting radiation of a low dose by means of a source of radiation.
Preferably the same source is used as the one used for recording the final radiation image so that the fluoroscopic image is taken from the same viewing angle as the viewing angle set when generating the radiation image.

Radiographic systems exist which are designed for recording a radiation image of a patient and can additionally be switched between a radiographic and a fluoroscopic irradiation mode, the fluoroscopic mode then being used for positioning.

US 2019/0183438 describes a method for patient positioning wherein following an examination request of a body region, the body region is pre-positioned in the radiography system for the radiographic recording. Next, at least one of a recording unit of the radiography system and an image detector of the radiography system are pre-positioned for the radiographic recording.
Then, a positioning recording of the body region is performed via the radiography system; the radiography system being switched into the fluoroscopy mode and the positioning recording being a fluoroscopy recording.
Positioning information is generated from the positioning recording and is outputted.

Even if the above system uses a fluoroscopic image to obtain position information, errors may still occur and new fluoroscopic images may still need to be taken when a new recording is to be performed.

Although the dose used for recording a fluoroscopic image is lower than the dose used for actual radiography recording, the patient is still subjected to a certain amount of radiation. Preferably this amount is kept as low as possible. So it is important to reduce or even eliminate the number of additional fluoroscopic images that is required.

Furthermore generating additional fluoroscopic images implies a longer occupation time of the radiographic image recording system which is to be avoided.

The present invention aims at optimizing methods as described higher for positioning a patient for radiographic image recording guided by a fluoroscopic image taken in the same positioning situation,

### SUMMARY OF THE INVENTION

The above-mentioned aspects are realized by a method as set out in claim 1.

The method of the invention is applied in an apparatus for positioning an object for a radiographic recording guided by a fluoroscopic positioning image as described below.

Further aspects and further embodiments of this invention are set out in the depending claims.

When in the context of the present invention reference is made to a patient, this can be a human patient or an animal.
The invention is likewise applicable to generating a radiation image of an object.

A body region is to be interpreted as a part of a body (human or of an animal), a bone, an organ etc. It can also be a region of an object.

The method of the present invention or parts thereof can be implemented as software modules to run on a computer.
The present invention can thus be implemented as a computer program product adapted to carry out all aspects of the method of the present invention when run on a computer. The invention also comprises a computer readable medium comprising computer executable program code adapted to carry out the steps of the method of the present invention.

Further advantages and embodiments of the present invention will become apparent from the following description and drawings.

### BRIEF DESCRIPTION OF THE DRAWINGS

- Fig. 1: illustrates generating a first radiation image,
- Fig. 2: illustrates the stitching of two radiation images.

### DETAILED DESCRIPTION OF THE INVENTION

The present invention will be described in detail below with reference to the drawings.

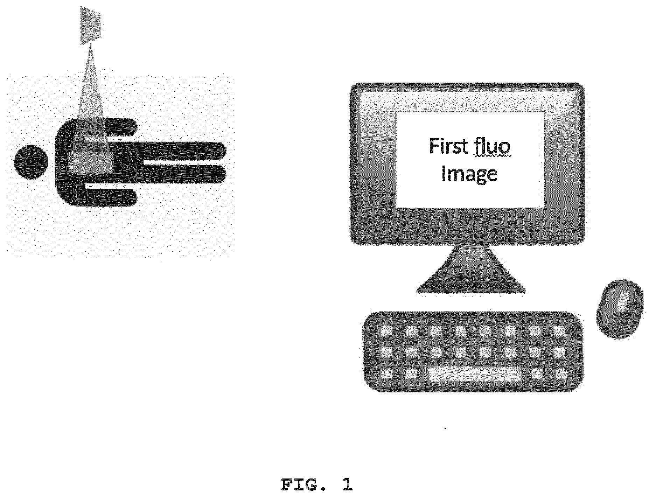

### Apparatus (Fig. 1)

The method of the present invention can be executed in a radiographic imaging system as described below.

The radiographic imaging system is a system for projection radiography.

It basically consists of a source of radiation for generating a bundle of x-rays and an associated control unit for setting the control parameters that determine the characteristics of the radiation bundle.

The system further comprises a radiation detector for detecting image-wise modulated x-rays and converting the image-wise modulated x-rays into a digital image which can be used to display an image or to generate a hard copy image.

The system also comprises a patient support holding a patient in a position for recording a radiation image (in some cases the patient support also holds the detector). In most cases this support is a table on which the patient lies during image recording.

The source of radiation is mounted in a radiology room and the source of radiation and the patient support can be moved relatively (translated, rotated, tilted) so that the area onto which radiation impinges when the source of radiation is activated can be adjusted by adjusting the relative position of the radiation soucre and the patient support.

The source of radiation is further commonly provided with a collimator which e.g. comprises a set of x-ray attenuating collimator blades that can be adjusted so as to reduce the size and shape of the radiation beam and consequentially can further delineate the region on the patient onto which the radiation emitted by the source of radiation impinges.

Furthermore the system comprises a visual camera, e.g. a 2D or 3D camera coupled to the source of radiation and arranged so that its visual field of view at least comprises the radiation field of the source of radiation.

In order to perform the method of the present invention the system also comprises means for generating a fluoroscopic image of the region that is envisaged to be irradiated by the source of radiation. The means for generating a fluoroscopic image of the region of interest comprise a source of radiation arranged to emit a fluoroscopic dose of radiation which his a lower dose than the dose emitted by the x-ray source for generating an x-ray image of the region of interest.

Preferably the same source of radiation is used for recording the radiation image as well as for generating the fluoroscopic image.
In this case a source of radiation is chosen that can be switched in between fluoroscopic mode and x-ray imaging mode. This embodiment is advantageous because the fluoroscopic image and the x-ray image will thus be taken from the same viewing angle without any adjustment being necessary.

The system also comprises a computer and associated storage means for controlling operation of the components of the radiographic imaging system and for storing information about the system or generated by the system such as information about the position of the patient and of the components of the imaging system.

### Method

The method of the present invention can be applied when more than one radiation exposure is considered (not necessarily also performed) of the same region or of another region within the field of view of a fluoroscopic image generated for the first region or of a second region which is at least partially overlapping with a first region for which a fluoroscopic image was generated and which second region thus falls at least partially outside the fluoroscopic image generated for the first region.

A workstation running a radiology information system can be coupled with the radiation image recording system.
If an examination request comprising patient identification data and data regarding the planned examination is generated for a specific patient, this request can be entered in the radiology information system (RIS) running on the workstation.
Alternatively these data can be entered manually or can even be entered through the use of (an) identification code(s) referring to patient data and / or examination data.

The examination request generally comprises a definition of the body region to be examined and occasionally also the data regarding the settings for the radiation exposure that is to be performed.

Settings corresponding with given examination type are generally predefined and stored by the workstation. They can be retrieved and sent to a radiographic imaging system in the radiography room.

Among these data are the source to patient distance and settings regarding the amount of radiation to be emitted.
Further settings are collimation settings, i.e. settings regarding the opening of a collimator for limiting the area that will be irradiated by the emitted bundle of radiation.

These data are available to the operator of the x-ray imaging system in the radiology room.

When the patient enters the radiology room he/she is positioned on a patient support.

Next the settings of the source of radiation are performed. For image recording the source of radiation is brought in a position that is optimal for the requested examination.

To guide this operation the system comprises a component for generating a fluoroscopic image.

In a preferred embodiment the radiography system is suitable for both projection radiography as well as fluoroscopy and can be switched between these two modes.

For the purpose of positioning the body region of interest for x-ray image recording the source of radiation is switched to a fluoroscopic mode (the fluoroscopic dose is lower than the dose applied for effective x-ray image recording) and at least one fluoroscopic image is generated.

The generated fluoroscopic image is displayed on a display screen coupled to a computer arranged to control the operation of the system and being visible to the operator of the radiology imaging system. The operator will examine the fluoroscopic image and will adjust the settings of the source of radiation (or of the patient or of the patient support). Adjustment is performed until a next fluoroscopic image shows the part of the patient a radiation image of which will be taken according to the examination request.

If the region which is visible on the fluoroscopic image matches the region specified in the examination request, the positioning is ready for the final radiographic image recording. The x-ray source may be activated to generate the radiographic image.

In the same position a visual image is generated of the patient by means of the visual 2D or 3D camera.

According to the present invention the generated fluoroscopic image(s) is/are stored together with the visual image(s) generated by the 2D or 3D visual camera and associated information such position information, table/wall stand, patient - source distance (SID), tube angle, collimator settings etc.

The position of the body region can be determined by means of its coordinates relative to a pre-defined reference position.

When a further (e.g. second) radiation image is to be generated of a region covering the same field of view or of a region which falls in the field of view of an already generated fluoroscopic image, the stored fluoroscopic image(s) and the associated position data are retrieved and are used to guide the patient positioning.

For a new radiation image the operator will relatively move the source of radiation and the patient support relatively so as to irradiate another body region.

As long the operator moves the patient support and radiation soure relatively or changes the collimator settings so that the new area falls within the already generate fluoroscopic image, the part that will be exposed can be deduced from the previously taken image which was (preferably) stored in a storage device associated with the computer controlling the radiation imaging system's operation and retrieved for the above-described purpose. This previously generated image is preferably the previously generated fluoroscopy image but could also be the previously generated radiographic image if available.

The calculations to be performed to identify the part that is going to be exposed can be done via the feedback from the SID, collimator, positioner information (tube angle etc.). In case no positioning information is available the generated visual image together with beam field information can also be used to identify the area which will be exposed during a next exposure in the new relative position.

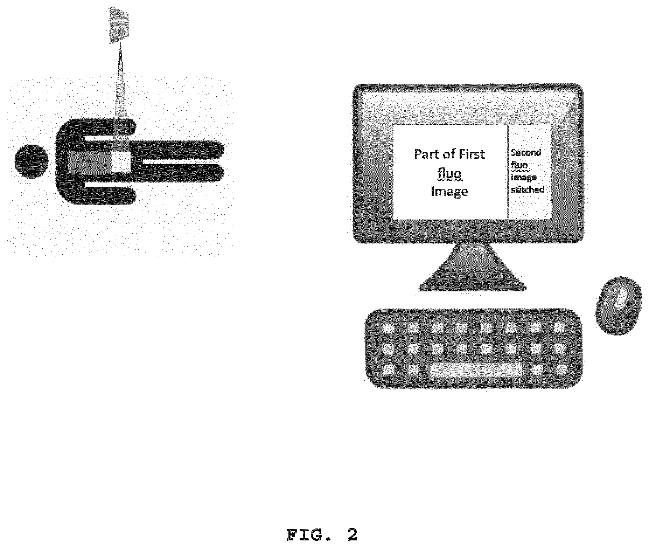

When an exposure is envisaged of a body region which does not entirely fall within the field of view registered in the fluoroscopic image corresponding with a first set up with a first relative position of the source of radiation and the patient, the first fluoroscopic image can still be used although it does not comprise the entire image required for appropriate patient positioning (Fig. 2).

In this case an additional fluoroscopic image is to be generated at least covering the region which falls outside the field of view of the first fluoroscopic image and which is still of interest for the second envisaged x-ray exposure.

From the moment on the operator moves outside the existing image, the collimator will be adapted that only the area is exposed where no image information is available.

The collimator can be positioned that only the not yet irradiated part of the body region is exposed. The image which will be displayed on the screen and shown to the operator (preferably on a display screen of a computer controlling the radiation imaging device) is then a combined image generated by stitching the first image and the second new partial image.
The already generated image can be the already generated fluoroscopy image or a radiographic image if available.

Having described in detail preferred embodiments of the current invention, it will now be apparent to those skilled in the art that numerous modifications can be made therein without departing from the scope of the invention as defined in the appending claims. ■

## Claims

1. Method for positioning a body region of a patient for a radiographic image recording by means of a radiographic image recording system comprising
- positioning a radiographic image recording system and the body region of the patient in a first relative position for a first radiographic recording of said region,
- generating a fluoroscopic image of the body region in said first relative position,
- for a second radiographic recording of a body region within the field of view recorded in said fluoroscopic image, relatively positioning said radiation image recording system and said body region so that said body region is irradiated by said source of radiation, said positioning being guided by said fluoroscopic image.

2. A method according to claim 1 wherein the position of the body region is recorded and compared with the position of the body region in the fluoroscopic image and wherein the relative position of the body region and the radiation image recording system is adjusted so that they match.

3. A method according to claim 1 wherein the position of the body region is recorded by taking a visual image of the body region to be irradiated during said second radiographic image recording.

4. A method according to claim 1 wherein the position of the body region is determined by means of its coordinates relative to a reference position.

5. A method according to claim 1 wherein the body region to be subjected to the second radiographic image is only partially recorded in said fluoroscopic image and wherein a second fluoroscopic image is generated of at least the part of the body region which is not recorded in the first fluoroscopic image and wherein first and second fluoroscopic images are stitched to generate a stitched image that is used for positioning the radiographic system for the second image recording.

6. A computer program comprising software code adapted to perform the method of any of the preceding claims when executed by a computer.

7. A computer readable storage medium comprising the computer program of any of claims 1 - 5.
